(19) Europäisches Patentamt
European Patent Office
Office européen des brevets

(11) EP 2 675 821 B1

(12) **EUROPEAN PATENT SPECIFICATION**

(45) Date of publication and mention of the grant of the patent:
**18.04.2018 Bulletin 2018/16**

(21) Application number: **12711108.6**

(22) Date of filing: **15.02.2012**

(51) Int Cl.:
***C07J 41/00*** *(2006.01)* ***C07J 75/00*** *(2006.01)*
***A61K 31/57*** *(2006.01)* ***A61P 25/00*** *(2006.01)*
***A61P 25/24*** *(2006.01)* ***A61P 25/28*** *(2006.01)*

(86) International application number:
**PCT/CZ2012/000016**

(87) International publication number:
**WO 2012/110010 (23.08.2012 Gazette 2012/34)**

(54) **PREGNANOLONE DERIVATIVES SUBSTITUTED IN 3ALPHA-POSITION WITH THE CATIONIC GROUP, USAGE AND PHARMACEUTICAL PREPARATION INVOLVING THEM**

IN 3ALPHA-POSITION SUBSTITUIERTE PREGNANOLONDERIVATE MIT EINER KATIONENGRUPPE, VERWENDUNG UND PHARMAZEUTISCHE ZUBEREITUNG DAMIT

DÉRIVÉS DE LA PRÉGNANOLONE, SUBSTITUÉS EN POSITION 3 ALPHA PAR LE GROUPE CATIONIQUE, PROCÉDÉS D'UTILISATION ASSOCIÉS, ET PRÉPARATIONS PHARMACEUTIQUES LES CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.02.2011 CZ 20110081**

(43) Date of publication of application:
**25.12.2013 Bulletin 2013/52**

(73) Proprietors:
- **Ústav Organické Chemie A Biochemie Akademie Ved Ceské Republiky, v.v.i.**
  **166 10 Praha 6 (CZ)**
- **Fyziologicky Ústav Akademie VED Ceské Republiky, V.V.I.**
  **142 20 Praha 4 (CZ)**

(72) Inventors:
- **CHODOUNSKÁ, Hana**
  **160 00 Praha 6 (CZ)**
- **KAPRAS, Vojtech**
  **170 00 Praha 7 (CZ)**
- **VYKLICKY, Ladislav**
  **251 68 Kamenice (CZ)**
- **BOROVSKÁ, Jirina**
  **250 82 Tuklaty (CZ)**
- **VYKLICKY, Vojtech**
  **251 68 Kamenice (CZ)**
- **VALES , Karel**
  **156 00 Praha 5 Zbraslav (CZ)**
- **STUCHLÍK, Ales**
  **255 88 Celákovice (CZ)**
- **RAMBOUSEK, Lukás**
  **742 21 Koprivnice (CZ)**

(74) Representative: **Herman, Vaclav**
**Hlavni 43**
**252 43 Pruhonice (CZ)**

(56) References cited:
**WO-A2-2009/108804 GB-A- 962 324**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

This invention relates to cationic steroid compounds, their applications and pharmaceutical compositions containing them. The invention particularly deals with pregnanolone derivatives substituted in 3alpha-position with the group bearing the cation, bound in this position. These derivatives may be beneficial in treatment of central nervous system (CNS) diseases, especially ischemic CNS injury, neurodegenerative alterations and diseases, depression, post-traumatic stress disorder and other stress-related disorders, schizophrenia and various psychotic diseases, pain, addiction, multiple sclerosis and autoimmune disorders, epilepsy, and gliomas as well as other CNS tumors.

### Background Art

Glutamate is the principal excitatory neurotransmitter in the central nervous system of mammals. During synaptic transmission, the post-synaptic responses occur via ionotropic and metabotropic glutamate receptors. Metabotropic receptors operate via G-proteins and mobilize calcium ions from intracellular compartments. Activation of ionotropic receptors results in increase in permeability of postsynaptic membrane for sodium, potassium and calcium cations by opening an ion channel, which is an integral part of the receptors.

Typical examples of ionotropic receptors are N-methyl D-aspartate (NMDA) receptors, AMPA and kainate receptors. Although current knowledge suggests specific role of various types of superfamily of glutamate receptors in the glutamate-induced excitotoxicity, ionotropic receptors are generally considered as key players in these processes. Activation of ionotropic receptors leads to alterations in intracellular concentrations of various ions, mainly of $Na^+$ and $Ca^{2+}$. Current research demonstrates that beside calcium, elevated intracellular levels of sodium ions can also lead to neuronal death. In neuronal cultures and in retina the activation of glutamate receptors may lead to damage even by sodium cation in absence of extracellular calcium ions. Nonetheless, toxicity of elevated glutamate levels is usually associated with elevations in intracellular concentrations of $Ca^{2+}$. Currently it is well established that there is a direct relationship between excessive influx of calcium into cells and glutamate-induced damage to neurons. Glutamate-induced pathological calcium elevation is usually ascribed to prolonged activation of ionotropic receptors. Elevation in intracellular calcium then may trigger the down-stream neurotoxicity cascade, which involves uncoupling of mitochondrial electron transport from ATP production, supranormal activation of enzymes such as calpain and other proteases, induction of specific protein kinases, NO-synthase, calcineurins and endonucleases. These changes may also promote the production of toxic reactive molecules such as reactive oxygen species (ROS) and induce changes in cytoskeleton architecture and activation of signals leading to apoptosis and mitochondrial damage (Villmann and Becker, 2007).

A number of preclinical studies show a remarkable ability of NMDA receptor antagonists to prevent from the excessive exocytose of glutamate and damage to the CNS. From the clinical point of view; however, their therapeutic potential is rather limited. Regarding the fact that glutamate receptors are ones of the most abundant in the CNS, application of their antagonists leads to wide variety of side effects, ranging from motor impairment to induction of psychotic symptoms. On the contrary, a large divergence of NMDA receptors and differences in their distribution at synapses and at extrasynaptic sites offer a possibility to search for drugs which selectively influence only a limited subset of NMDA receptors and thus to avoid the induction of unexpected side effects, while retaining their therapeutic neuroprotective activity.

Previous results demonstrated that naturally occurring 3α5β-pregnanolone sulfate affects the activity of NMDA receptor by a use-dependent manner. Therefore, this molecule has a more pronounced inhibitory action on the tonically activated NMDA receptors than on those phasically activated by glutamate during synaptic transmission. It was also demonstrated that activation of extrasynaptic tonically activated NMDA receptors is very important for excitotoxic action of glutamate (Petrovic et al., 2005).

Therefore, we have started the development and testing of novel NMDA receptor antagonists derived from neurosteroids. These newly synthesized drugs exhibit affinity to extrasynaptic NMDA receptors. Importantly, previous electrophysiological studies showed that these compounds bound preferentially to open NMDA receptor channels. Our compounds lack affinity for other types of receptor; so we could presume that they will not affect signal transmission between neurons. The suggested mechanisms of their action are the blockade of extrasynaptic tonically activated NMDA receptors and prevention of excessive action of glutamate on neurons.

In the last decade, the biomedical research focused on the study of the role of neurosteroids in the pathogenesis of number of neuropsychiatric diseases and evaluation of their therapeutic potential. Mechanisms of action of neurosteroids are conventionally associated with their activity on NMDA and GABA-A receptors. A number of experimental studies with animal models show their potential in therapy of several diseases of CNS, including neurodegenerative disorders, multiple sclerosis, affective disorders, alcoholism, pain, insomnia or schizophrenia (Morrow, 2007; Weaver, 2000).

Neurosteroids also play a crucial role in the regulation of reactivity to stress and stress-related CNS disorders. Corticosteroid levels are known that acutely increase after exposition to a stressor; this represents an adaptive mechanism. On the other hand, experimental models of chronic stress and depression in laboratory rodents show decreased levels of neurosteroids both in brain and in plasma. Similar findings are often reported in.patients suffering from depressions and pre-menstruation syndrome suggesting impairments in the CNS homeostatic mechanisms in stress-related neuropsychiatric disorders.

Steroid compounds affect activity and plasticity of neural and glial cells during early life, and later in development they play an essential neuroprotective role in the adult CNS. Steroids are released by sexual and adrenal glands as well as in the CNS. Steroids secreted by peripheral glands reach brain, medulla and spinal cord via blood circulation. Nonetheless, some neural steroids (i.e., neurosteroids) are biosynthesized directly in the CNS. The most studied neurosteroids are pregnenolone, progesterone, dehydroepiandrosterone (DHEA), their reduced metabolites, and esters. Not much is known about regulation of neurosteroid synthesis in the CNS, but it is generally assumed that they may underlie interaction of multiple cell types in the CNS. Synthesis of progesterone by Schwann cells, surrounding peripheral nerves, is regulated by signals diffusing from neurons.

Neurotrophic and neuroprotective properties of some neurosteroids were convincingly demonstrated both in cultures and *in vivo.* Progesterone plays a pivotal role in neurological recovery from traumatic brain and spinal cord injury by mechanisms including protection against excitotoxic damage to the brain, lipid peroxidation and by induction expression of specific enzymes. For example, after cutting the spinal cord, this steroid increases the number of NO-synthase-expressing astrocytes in place adjacent to cut both in the distal and proximal segment of the cord.

This steroid was also shown to regulate formation of new myelin sheaths. This fact was shown in regenerating rat *sciatic* nerve in the culture with sensory neurons and Schwann cells. Progesterone also supports myelination by activation of genes coding for proteins participating in this process.

As mentioned before, neurosteroids importantly modulate the function of membrane receptors for various neurotransmitters, namely $GABA_A$ receptors, NMDA receptors and sigmal-opioid receptors. These mechanisms are most likely responsible for psychopharmacological effects of steroids and may at least partly account for their anticonvulsant, anxiolytic, neuroprotective and sedation effects as well as for their influence upon learning and memory functions. For instance, pregnanolone sulfate was shown to be capable of reversing cognitive deficit in aged animals and exerting a protective effect on memory in several amnesia models. Recent studies have demonstrated direct effect of neurosteroids on intracellular receptors. Despite absence of direct evidence for binding of neurosteroids to corticoid receptors, they may obviously modulate their function *indirectly*, by interaction with protein kinases C and A, MAP-kinase (MAPK) or CaMKII. Moreover, pregnanolone and pregnanolone sulfate were shown to affect microtubule-associated proteins and increase the rate of microtubule polymeration, which may in turn affect neuronal plasticity.

Sulfated esters of neurosteroids also play a physiological role in the regulation of receptors for excitatory and inhibitory neurotransmitters and participate in the natural protective properties of CNS tissue. Sulfated esters of neurosteroids and their analogs are promising molecules, potentially beneficial for treatment of CNS disorders. Nonetheless, a ratio between neurosteroids and their sulfated esters is maintained enzymatically in the CNS tissue *in vivo.* Exogenous administration of sulfated esters may not lead to improvement in the protective functions, due to enzyme activity. The invented molecules are metabolically more stable analogs of sulfated esters of neurosteroids. Sulfated and thus polar steroids compounds generally do not penetrate the blood-brain, but it was demonstrated that intravenously administered pregnanolone sulfate reach the brain (Wang et. al., 1997). However, the ratio of sulfated and free steroid in the brain is stable. The transport of sulphated analogs is probably mediated by active exchange mechanisms associated with so-called *organic anion transport protein (OATP),* which is expressed in the cells of brain tissue.

Advantage of our molecules is that they retain similar pharmacological and physiological properties as pregnanolone sulfate, but they are not metabolically deactivated by sulfatases into non-conjugated metabolites.

WO 2009/108804 (University Emory) disclosd steroid analogues functionalized with polar substituents at the C3 and/or C20 positions of the steroid ring system that exhibit improved water solubility. The polar substituents at C3 position are amino acids bearing a free amino group containing a basic nitrogen atom with a lone pair (two electrons paired but not used in chemical bonding).

GB 962324 (ETABLISSEMENTS CLIN-BYLA) disclosd 3α-amino-5β -pregnane-20-one and its activity to central nervous system, but it did not disclose salts of above mentioned compound having in position C3 a tertiary ammonium group bearing alkyl or alkenyl groups with 1-18 carbon atoms in a straight or a branched carbon chain.

The present invention does not aim at amine substituent at C3 position, but rather at quaternary ammonium and/or guanidyl group, ie. permanently charged polyatomic ions, which are not dependent on the pH of their solution. Therefore it describes new structures of water insoluble compounds bearing at C3 such a substituent which determines their biological activity.

**Description of the Invention**

The present invention relates to compounds of general formula I,

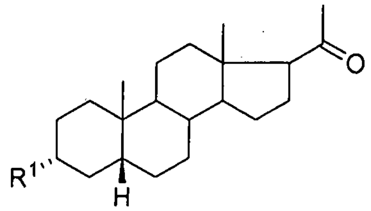

(I),

in which $R^1$ represents the group of general formula $R^3$-$R^2$-C($R^{13}$)-$R^{14}$-, where $R^2$ means $(CH_m)_{n}$- group, wherein m is 0 to 2, n is 1 to 18 and forms straight chain, which may be additionally substituted by one or more halogen atoms or primary, secondary or tertiary amino group, which may be either free or in the case of primary amino group protected by a removable protecting group, chosen from tert.butoxycarbonyl, trityl, benzyloxycarbonyl, 9-fluorenylmethoxycarbonyl, or *p*-nitrobenzyloxycarbonyl,

$R^3$ represents cationic group, chosen from guanidyl group of general formula (a)

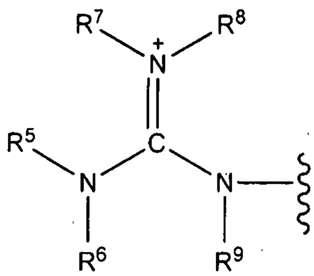

(a)

or ammonium group of general formula (b)

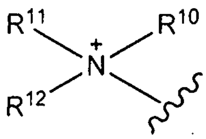

(b)

where $R^5$- $R^{12}$ are hydrogen atoms or alkyl or alkenyl groups with 1 to 18 carbon atoms in a straight or a branching carbon chain, $R^{13}$ is chosen from a group, involving oxygen, nitrogen or sulfur atom bound by a double bond to carbon, or $R^{13}$ are two hydrogen atoms;

$R^4$ is bivalent or multivalent atom, chosen from oxygen, nitrogen or carbon atom and in case where $R^4$ is multivalent atom, that is carbon or nitrogen, its additional valence(s) is (are) hydrogen(s) and any of hydrogen atoms may be substituted by alkyl or alkenyl having from 1 to 4 carbon atoms.

In one aspect of the invention pregnanolone derivatives, substituted in the 3alpha-position with the cationic group are of general formula I described above, where the substiuent $R^1$ = ammonium group of general formula (b) described above and $R^{10}$ - $R^{12}$ of this group are alkyl or alkenyl groups with 1 to 18 carbon atoms in a straight or a branched carbon chain.

Presented invention also includes pregnanolone derivatives substituted in position 3alpha with cationic group of general formula I, for usage for treatment of neuropsychiatric disorders related to dysbalances of glutamatergic neurotransmitter system, as ischemic CNS injury, neurodegenerative changes, and disorders of CNS, mood disorders, depression, post-traumatic stress disorder, and other stress-related disorders, anxiety, schizophrenia and other psychotic illnesses, pain, addiction, multiple sclerosis, epilepsy, and gliomas.

The object of invention is also pharmaceutical preparation containing pregnanolone derivatives substituted in position 3alpha with cationic group of general formula I as active component.

The object of invention is pharmaceutical preparation containing as active substance the pregnanolone derivatives substituted in position 3alpha with cationic group of general formula I, for use in the treatment of neuropsychiatric disorders related to dysbalance of glutamatergic neurotransmitter system as ischemic CNS injury, neurodegenerative changes, and disorders of CNS, mood disorders, depression, post-traumatic stress disorder, and other stress-related disorders, anxiety, schizophrenia, and other psychotic illnesses, pain, addiction, multiple sclerosis, epilepsy and, gliomas.

The object of invention is finally pharmaceutical preparation containing as active substance the pregnanolone derivatives substituted in position 3alpha with cationic group of general formula I for production of standards of neuroactive steroids utilized in experimental research, analytic chemistry. Moreover also utilization of these compounds as active substances contained in dietary supplements or cosmetic preparations designated for improvement of reactions of particular parts of organisms on higher stress, namely oxidative, nutrient, or caused by free radicals, or caused by ageing.

The invention is based on the results of experiments, in which we studied the activity of pregnanolone sulfate on native and recombinant NMDA receptors. These experiments showed that the naturally occuring neurosteroid inhibits responses elicited by exogenous application of specific agonists of NMDA receptors. We have prooved, that pregnanolone sulfate binds only to activate receptors (use-dependent activity) but does not bind to the ionic channel as some substances of $Mg^{2+}$ , ketamine, dizocilpine or memantine type. The rate of binding and the mechanism of action pregnanolone sulfate causes higher inhibitory effect on glutamate tonically activated receptors, than on phasically activated receptors during the synaptic trasmission. It was newly discover that analogues synthesized by us, which are the object of the invention, showe the same mechanism of action on the NMDA receptors.

Application of pregnanolone sulfate does not cause improvement of function studied as consequence of enzymatic activity, but molecules presented here constitute better analogues, which are not hydrolysable by sulfatases.

Various structural modifications of our invented compounds of general formula I have shown only minimal differences in their biological activity; these findings are congruent with previous electrophysiological results using patch-clamp technique and assessing binding kinetics of these compounds on the NMDA receptors.

The data confirm capability of NMDA receptor antagonists to prevent the excessive release of glutamate and subsequent damage of the CNS leading to deterioration of behavior. From the clinical point of view; however, their therapeutic potential is rather limited regarding the fact that their application leads to wide variety of side effects, ranging from motor impairment to induction of psychotic symptoms.

Main advantage of 3$\alpha$C-substituted analogs of pregnanolone, use-dependent NMDA antagonists, constitutes absence of serious side effect typical for competitive NMDA antagonists, while retaining their therapeutic activities.

**Description of figures on drawings:**

Fig. 1A presents current response elicited by 1 mmol.$l^{-1}$ of glutamic acid and reversible inhibition of the current response caused by the compound from the example 2, which was applied in the concentration 10 $\mu$mol.$l^{-1}$ simultaneously with the glutamic acid in time intervals presented by empty rectangless. The membrane potentials were maintained at -60 mV or +60 mV. Patch-clamp technique was used to record the currents from cutivated HEK293 cells with transfected NR1/NR2B NMDA receptors. Relative inhibition elicited by the compound from the example 2 was calculated from the formula (1-a/b)* 100 (%). Fig. 1B is the diagram showing an independence of average inhibition elicited by the compound from the example 2 (10 $\mu$mol.$l^{-1}$) in time intervals presented by empty rectangless depicted on the axis y, on the maintained membrane potential depicted on the axis x.

Fig. 2 presents an effect of pregnanolone arginate on spontaneous locomotor activity in the open-field test. Total path length in metres is depicted on the axis y, first column responds to intact animals, middle column responds to control animals injected with saline and cyclodextrin, third column responds to animals injected with pregnanolone argininate.

Fig. 3 presents an effect of pregnanolone arginate and pregnanolone nitrate on locomotion and cognitive function in Carousel maze. The total distance (m) per session in the AAPA training is depicted on the axis y. Control animals obtained cyklodextrine, dizocilpine (MK) was used as a positive control.

Fig. 4 presents a number of entrances into shock sector, related to cognitive functions of animals influenced by of pregnanolone arginate and/or pregnanolone nitrate. The number of entrances is ploted on the axis y. Control animals obtained cyklodextrine, dizocilpine (MK) was used as a positive control; * denotes $p < 0.05$, *** denotes $p<0.001$ compared to cyclodextrin controls.

## Examples

List of abbreviations

| | |
|---|---|
| DMSO | dimethylsulfoxide |
| DMAP | 4-dimethylaminopyridine |
| DCC | dicyklohexylkarbodiimid |
| DMF | dimethylformamide |
| HRMS | high resolution mass spectrometry |
| Boc | *tert*-butoxycarbonyl |
| Pbf | 2,2,4,6,7-pentamethyl-2,3-dihydrobenzofuran-5-sulfonyl |
| m | multiplet |
| bm | broad multiplet |
| d | dublet |
| t | triplet |
| EI | electron impact ionozation |
| ESI | electrospray ionization |
| eq. | equivalent |
| IR | infrared spectroscopy |
| MS | mass spectroscopy |
| NMR | nuclear magnetic resonance |
| Et | ethyl |
| *t*-Bu | tertiary butyl |
| Ac | acetyl |
| HEK | human embryonic kidney cells |

| | |
|---|---|
| GFP | green fluorescent protein |
| $IC_{50}$ | the half maximal inhibitory concentration |
| Opti-MEM® I | minimum essential media, Invitrogen's product |
| DHEA | 5-dehydroepiandrosterone |
| EGTA | ethylene glycol tetraacetic acid |
| EDTA | ethylene diamine tetraacetic acid |
| HEPES | 4-(2-hydroxyethyl)-1-piperazineethanesulfonic acid |

Biological activity on cell cultures

Degree of activated NMDA receptor inhibition by steroid cationic compounds was measured *in vitro* electrophysiologically on cultivated HEK293 cells (Human Embryonic Kidney 293 cells) 24-48 h after the transfection with DNA plasmids, coding NR1-1a and NR2B subunit of NMDA receptor. Transfected cells were identified by means of fluorescent green protein (GFP) fluorescence. Its genus was transfected together with the both receptor subunit genes.

Steroid-containing solutions were prepared from fresh solution (20 $mmol.l^{-1}$, of steroid dissolved in dimethylsulfoxide, DMSO), which was added to the extracellular solution containing 1 $mmol.l^{-1}$ glutamic acid and 10 $\mu mol.l^{-1}$ of glycine. Same concentrations of DMSO were added to all other extracellular solutions.

Current responses produced by extracellular application of glutamic acid solution (1 $mmol.l^{-1}$) were measured from the whole cell by *patch-clamp* technique, which is used for the study of transport of charged particles through model and also natural biological membranes. The currents were measured at membrane potential maintained at -60 mV and +60 mV. Steroid compounds studied lowered response amplitude elicited by glutamic acid. Application of 10 $\mu mol.l^{-1}$ steroid solution the mean inhibition effect reached 65 -70%. It can be compared with 100 $\mu mol.l^{-1}$ of endogenous neurosteroid 5β-pregnanolon-3α-yl-sulfate, which inhibited responses elicited by NMDA receptor to 67%.

### Example 1

**20-Oxo-5β-pregnan-3α-yl (2*S*)-2-(benzyloxycarbonylamino)-5-(3-nitroguanidino)pentanoate**

An oven-dried 100 mL flask with magnetic stir bar was charged with the mixture of compound II (300 mg, 0.94 mmol), $N_{\alpha}$-(carbonyloxybenzyl)-$N_{\omega}$-nitro-L-arginine (366 mg, 1.03 mmol) and DMAP (12 mg; 0.09 mmol). 20 mL of dry acetonitrile were added under the argon atmosphere and resulted mixture was cooled in ice bath. 1 $mol.l^{-1}$ solution of DCC in benzene (1.41 mL; 1.41 mmol) was then added dropwise to the stirred cold mixture. The cooling bath was then

removed and the reaction mixture was stirred for 16 hrs. The reaction as quenched with saturated solution of $NaHCO_3$ (50 mL), extracted with EtOAc (3x25 mL), washed with brine (50 mL), dried with anhydrous $MgSO_4$ filtered and evaporated *in vacuo.* N,N'-Dicyclohexylurea was crystallized from minute amount of acetone and filtered off. Filtrate was concentrated *in vacuo* again and loaded in toluene on a column of silica (30 g). Elution with a mixture of hexanes:acetone (7:3) and subsequent evaporation of solvents gave 20-oxo-5β-pregnan-3α-yl (2*S*)-2-(benzyloxycarbonylamino)-5-(3-nitroguanidino)pentamoate as white foam (473 mg; 77%).

$^1$H NMR (500 MHz, $CDCl_3$) δ 7.41-7.31 m (5H, Ph), 5.67 (d, 1H, J = 8.0 Hz, NHCbz), 5.12 (s, 2H, C$H_2$-Ph), 4.84-4.74 (m, 1H, 3-CH), 4.37-4.28 (m, 1H, 2'-CH), 3.63-3.52 (m, 1H, 5'a-CH), 3.35-3.23 (m, 1H, 5'b-CH), 2.57 (t, 1H, J = 9.0 Hz), 2.12 (s, 3H, 21-$CH_3$), 0.94 (s, 3H, 19-$CH_3$), 0.60 (s, 3H, 18-$CH_3$).

$^{13}$C NMR (101 MHz, CDCl3) δ 209.54, 171.34, 159.24, 135.94, 128.70, 128.52, 127.77, 76.34, 67.59, 63.80, 56.60, 52.27, 44.32, 41.87, 40.45, 40.22, 39.09, 35.80, 34.88, 34.61, 32.09, 31.67, 31.51, 26.88, 26.51, 26.25, 24.41, 24.33, 23.22, 22.93, 20.88, 13.42.

IR ($CHCl_3$): 3397 (NH), 1729 (C=O, ester), 1702 (C=O, ketone), 1626, 1606 (C=NH), 1515 (carbamate), 1387 ($CH_3$), 1348 ($NO_2$), 1291, 1277 (CO), 1232 (carbamate), $cm^{-1}$.

ESI m/z 654.1 (45%, $[M+H]^+$), 676.3 (100%, $[M+Na]^+$); HRMS-ESI m/z 654.3861 ($[M+H]^+$, C35H52O7N5 requires 654.3861).

### Example 2

### 20-Oxo-5β-pregnan-3α-yl L-argininate diacetate salt

Compound from the example 1 (216 mg; 0.33 mmol) was dissolved in a mixture of acetic acid (0.5 mL) and methanol (9.5 mL). 10% Pd on carbon (44 mg) was added and reaction mixture was stirred at r.t. under atmospheric pressure of hydrogen gas for 72 hrs. Catalyst was filtered off through pad of diatomaceous earth and the solvents were evaporated. Chromatography on silica gel (4 g) in MeOH:AcOH:$H_2O$ afforded 20-Oxo-5β-pregnan-3α-yl L-argininate diacetate salt as an off-white foam (187 mg; 95%).

$^1$H NMR (500 MHz, $CDCl_3$:$d_4$-MeOD, 4:1) δ 4.82-4.74 m (m, 1H, 3-CH), 3.47 (t, 1H, J = 6.4 Hz, 2'-CH), 3.15 (t, 2H, J = 6.8 Hz, 5'-CH), 2.58 (t, 1H, J = 8.8 Hz, 17-CH), 2.17 (s, 3H, 21-$CH_3$), 2.01 (s, 6H, -$OOCCH_3$), 0.96 (s, 3H, 19-$CH_3$), 0.61 (s, 3H, 18-$CH_3$).

$^{13}$C NMR (101 MHz, $d_4$-MeOD) δ 212.41, 169.93, 78.62, 65.01, 58.03, 53.89, 45.55, 43.44, 41.95, 41.85, 40.38, 37.34, 36.11, 35.92, 33.36, 31.72, 28.94, 28.19, 27.75, 27.67, 25.80, 25.57, 24.07, 23.81, 22.13, 13.88.

IR (KBr): 3342, 3267, 3167 ($NH_3^+$), 1726 (C=O, ester), 1699 (C=O, ketone + guanidinium), 1679, 1601, (guanidinium), 1551, 1408 ($AcO^-$), 1387 ($CH_3$), 1364 ($COCH_3$), 1235, 1167 (CO), $cm^{-1}$.

ESI m/z 475.3 (100%, $[M-2AcOH+H]^+$); HRMS-ESI m/z 475.3640 ($[M-2Cl+H]^+$, C27H47O3N4 requires 475.3643).

### Example 3

### 20-Oxo-5β-pregnan-3α-yl 2-[(tert-butoxycarbonyl)amino]-5-(3-{(2,2,4,5,7-pentamethyl-2,3-dihydrobenzofuran-6-yl)sulfonyl)guanidino}pentanoate

In an oven-dried 100 mL flask with magnetic stir bar was charged with the compound II (500 mg, 1.57 mmol), Boc-L-Arg(Pbf)-OH (994 mg, 1.88 mmol), dimethylaminopyridine (DMAP, 21 mg; 0.16 mmol) and flushed with argon. Solids were dissolved in dry benzene (45 mL) and the reaction mixture was cooled in ice bath. 1 $mol.l^{-1}$ solution of dicyclohexylcarbodiimide (DCC) in benzene (1 $mol.l^{-1}$, 1.41 mL; 1.41 mmol) was then added dropwise. The reaction mixture was heated up to r.t. and stirred for 16 hrs. The reaction was quenched with saturated solution of $NaHCO_3$ (50 mL). Product was extracted with EtOAc (3x25 mL), washed with brine (50 mL), dried with anhydrous $MgSO_4$ filtered and evaporated *in vacuo.* N,N'-Dicyclohexylurea was crystallized from minute amount of acetone and filtered off. Filtrate was concentrated *in vacuo* again and loaded in toluene on a column of silica (50 g). Elution with hexanes:acetone (7:3) and subsequent evaporation of solvents gave 20-Oxo-5β-pregnan-3α-yl 2-((tert-butoxycarbonyl)amino)-5-(3-((2,2,4,5,7-pentamethyl-2,3-dihydrobenzofuran-6-yl)sulfonyl)guanidino)pentanoate as white foam (1.29 g; 99%).

$$[\alpha]_D = +53.0\ (c\ 0.234);$$

$^1$H NMR (500 MHz, $CDCl_3$) δ 6.31 (bm, 1H, guanidine), 6.04 (s, 2H, guanidine), 5.30 (d, 1H, J = 7.7 Hz, NHBoc), 4.82-4.74 (m, 1H, 3-CH), 4.25-4.19 (bm, 1H, 2'-CH), 3.40-3.30 (m, 1H, 5'a-CH), 3.25-3.15 (m, 1H, 5'b-CH), 2.96 (s, 2H, $CH_2$), 2.59 (s, 3H, $CH_3$), 2.53 (s, 3H, $CH_3$), 2.53 (t, 1H, J = 9.0 Hz), 2.10 (s, 3H, 21-$CH_3$), 1.46 (s, 3H, 2x$CH_3$), 1.43 (s, 9H, tBu), 0.94 (s, 3H, 19-$CH_3$), 0.59 (s, 3H, 18-$CH_3$).

$^{13}C$ NMR (101 MHz, CDCl3) δ 209.55, 171.81, 158.70, 156.01, 138.37, 133.08, 132.35, 124.54, 117.42, 86.31, 80.49, 75.94, 63.79, 56.57, 44.29, 43.29, 41.88, 40.89, 40.41, 39.10,
35.80, 34.92, 34.62, 32.13, 31.49, 28.59, 28.35, 26.89, 26.57, 26.26, 24.39, 23.23, 22.91, 20.87, 19.21, 17.84, 13.40, 12.45.
IR ($CHCl_3$): 3432, 3345 (NH), 1728 (C=O, ester), 1699 (C=O, ketone), 1633, 1623, 1559(guanidin), 1506 (NHBoc), 1408 (guanidin), 1393 (tBu), 1385, 1370 ($CH_3$), 1358 ($COCH_3$), 1158 ($SO_2$), $cm^{-1}$.
ESI m/z 827.5 (63%, $[M+H]^+$), 849.5 (100%, $[M+Na]^+$); HRMS-ESI m/z 827.49907 ($[M+H]^+$, C45H71O8N4S requires 827.49871).

For C45H70N4O8S (827.1) calculated: 65.34% C, 8.53% H, 6.67 % N, 3.88% S; found: 65.51% C, 8.68% H, 6.43% N, 3.70 % S.

**Example 4**

**20-Oxo-5β-pregnan-3α-yl L-argininate, dihydrochloride salt**

Compound from the example 3 (430 mg; 0.52 mmol) was dissolved in neat trifluoroacetic acid (0.5 mL) and stirred for 48 hrs at r. t. The reaction mixture was then poured into saturated solution of $NaHCO_3$ (50 mL), extracted with chloroform (4x20 mL), washed with 5% solution of HCl (50 mL), dried with anhydrous $Na_2SO_4$, filtered and evaporated under the reduced pressure. Crystallization in chloroform afforded 20-oxo-5β-pregnan-3α-yl L-argininate dihydrochloride salt as white crystals (225 mg; 79%);

$$[\alpha]_D = +71.4 \text{ (c 0.224; MeOH)};$$

$^1H$ NMR (500 MHz, $d_4$-MeOD) δ 4.92-4.88 m (1H, 3-CH), , 4.84-4.74 (m, 1H, 3-CH), 4.06 (t, 1H, J = 6.4 Hz, 2'-CH), 3.27 (t, 2H, J = 6.8 Hz, 5'-CH), 2.64 (t, 1H, J = 8.8 Hz, 17-CH), 2.12 (s, 3H, 21-$CH_3$), 0.99 (s, 3H, 19-$CH_3$), 0.61 (s, 3H, 18-$CH_3$).
$^{13}C$ NMR (101 MHz, $d_4$-MeOD) δ 212.41, 169.93, 78.62, 65.01, 58.03, 53.89, 45.55, 43.44, 41.95, 41.85, 40.38, 37.34, 36.11, 35.92, 33.36, 31.72, 28.94, 28.19, 27.75, 27.67, 25.80, 25.57, 24.07, 23.81, 22.13, 13.88.
IR (KBr): 2935 ($NH_3^+$), 1744 (C=O, ester), 1706 (C=O, ketone), 1667, 1652, 1625 (guanidinium), 1385 ($CH_3$), 1358 ($COCH_3$), 1226, 1193 (CO), $cm^{-1}$.
ESI m/z 475.4 (100%, $[M-2Cl+H]^+$); HRMS-ESI m/z 475.36398 ($[M-2Cl+H]^+$, C27H47O3N4 requires 475.36427).
For C27H48Cl2N4O3 (547.6) calculated: 59.22% C, 8.84% H, 12.95% Cl, 10.23 % N; found: 58.95% C, 8.72% H, 13.11% Cl, 9.99 % N.

**Example 5**

**20-Oxo-5β-pregnan-3α-yl 2-(methylguanidino) acetate hydrochloride**

Anhydrous creatine (59 mg, 0.5 mmol] was dissolved in tetrahydrofuran (15 ml), then the compound II (160 mg, 0.5 mmol), anhydrous magnesium sulfate (2 g) and catalytic amount of sulfuric acid was added. The mixture was stirred 16 h at the room temperature. Then it was poured into the ice water and product extracted into the chloroform (4 x 20 ml), organic layer was washed by cold solution of sodium hydrogen carbonate (5%), diluted hydrochloric acid (5%, 20 ml), dried by anhydrous sodium sulfate. The mixture was filtrated and solvents evaporated under the reduced pressure. Crystallization from chloroform afforded 20-oxo-5β-pregnan-3α-yl 2-(methylguanidino) acetate hydrochloride (107 mg; 47%).
$^1H$ NMR (500 MHz, $d_4$-methanol) δ 4,92-4.88 m (1H, 3-CH), 4,79 (d, 1H, 3-CH), 2,57 (t, 1H, J = 8,8 Hz, 17-CH), 2,12 (s, 3H, 21-$CH_3$), 0,99 (s, 3H, 19-$CH_3$), 0;61 (s, 3H, 18-$CH_3$).
IR (KBr): 2935 ($NH_3^+$), 1744 (C=O, ester), 1706 (C=O, keton), 1667, 1652, 1625 (guanidinium), 1385 ($CH_3$), 1358 ($COCH_3$), 1226, 1193 (CO), $cm^{-1}$.
ESI m/z 418.3 (100%, $[M-Cl+H]^+$); HRMS-ESI m/z 418.3073 ($[M-Cl+H]^+$, for $C_{24}H_{40}O_3N_3$ calculated 418,3070).

**Example 6**

**20-Oxo-5β-pregnan-3α-yl 4-(trimethylammonium) butanoate chloride**

3-Carboxy-N,N,N-trimethylpropan-1-ammonium chloride (prepared according to Lindstedt and Lindstedt, 1965, 69 mg; 0.38 mmol) was suspended in anhydrous $CH_2Cl_2$ (1 mL) under argon. The reaction flask was cooled in ice bath and oxalyl chloride (0.5 mL; 5.82 mmol) was added dropwise, followed by catalytic amount of dry DMF (3 μL; 0.03 mmol).

The heterogeneous mixture was then brought to r.t. and stirred for 16 hrs, during which all the solids dissolved. The mixture was evaporated under the reduced pressure and solid residue was dissolved in dry nitromethane (2 mL) and dry pyridine (0.10 mL; 1.24 mmol) under argon. Compound II (100 mg; 0.31 mmol) was added to this reaction mixture, which was then stirred for 4 hrs. Reaction was quenched with water (10 mL) and acidified to pH 4 with 5% aq. HCl. Product was extracted with $CHCl_3$ (3 x 20 mL), solution was washed with brine (10 mL, dried with anhydrous $MgSO_4$ and evaporated under the reduced pressure. Trituration with benzene removed the unreacted starting steroide II and the remaining product was subsequently crystallized from $CHCl_3$ : *n*-heptane (1:1) to give needle-like crystals (134 mg; 89%).

$$[\alpha]_D = +88.4\ (c\ 0.243);$$

$^1$H NMR (500 MHz, $CDCl_3$) $\delta$ 4.76-4.68 (m, 1H, 3-CH), 3.73-3.73 (bm, 2H, 4'-$CH_2$), 3.47 (s, 9H, $NCH_3$), 2.55 (t, 1H, J = 9.0 Hz, 17-CH), 2.49 (t, 2H, J = 6.2 Hz, 2' -$CH_2$), 2.12 (s, 3H, 21-$CH_3$), 0.94 (s, 3H, 19-$CH_3$), 0.60 (s, 3H, 18-$CH_3$).
$^{13}$C NMR (101 MHz, $CDCl_3$) $\delta$ 209.47, 171.49, 75.20, 65.61, 63.79, 56.62, 53.45, 44.26, 41.83, 40.41, 39.13, 35.76, 34.96, 34.59, 32.19, 31.46, 30.27, 26.87, 26.59, 26.24, 24.37, 23.22, 22.89, 20.82, 18.46, 13.38.
IR ($CHCl_3$): 2956 ($NMe_3^+$), 1722 (C=O, ester), 1699 (C=O, ketone), 1478 ($NMe_3^+$) 1386 ($CH_3$), 1360($COCH_3$), 1230 ($NMe_3^+$), 1188 (CO), $cm^{-1}$.
ESI m/z 446.6 (100%, $[M-Cl]^+$); HRMS-ESI m/z 446.3624 ($[M-Cl]^+$, C28H48O3N requires 446.3629).
For C28H48ClNO3 (482,1) calculated: 69.75% C; 10.03% H, 7.35% Cl, 2.91% N; found: 69.59% C, 9.99% H, 7.12 % Cl, 2.82% N.

**Example 7**

**20-Oxo-5β-pregnan-3a-yl 4-(trimethylammonium) hexanoate chloride**

From 5-carboxy-N,N,N-trimethylpropan-1-aminium chloride (210 mg, 1 mmol) by similar procedure as in example 6 was prepared 20-oxo-5β-pregnan-3α-yl-4-(trimethylamonium) hexanoate hydrochloride (101 mg; 76%).

$$\text{m.p.} = 205.5\text{-}207.5^\circ\text{C (decomposition)}$$

$$[\alpha]_D = +87.7\ (c\ 0.204)$$

$^1$H NMR (500 MHz, $CDCl_3$) $\delta$ 4,74-4,67 (m, 1H, 3-CH), 3,71-3,53 (bm, 2H, 5'-$CH_2$), 3,45 (s, 9H, $NCH_3$), 2,56 (t, 1H, J = 8.9 Hz, 17-CH), 2,49 (t, 2H, J = 6,1 Hz, 2'-$CH_2$), 2,12 (s, 3H, 21-$CH_3$), 0,95 (s, 3H, 19-$CH_3$), 0,61 (s, 3H, 18-$CH_3$).
$^{13}$C NMR (101 MHz, $CDCl_3$) $\delta$ 209.60, 172.65, 74.30, 66.50, 63.78, 56.59, 53.27, 44.26, 41.77, 40.33, 39.11, 35.72, 34.96, 34.57, 34.08, 32.20, 31.50, 26.85, 26.62, 26.24, 25.64, 24.36, 24.32, 23.23, 22.89, 22.82, 20.78, 13.37.
IR ($CHCl_3$): 2958 ($NMe_3^+$), 1720 (C=O, ester), 1700 (C=O, keton), 1478 ($NMe_3^+$) 1386 ($CH_3$), 1360 ($COCH_3$), 1231 ($NMe_3^+$), 1187 (CO), $cm^{-1}$.
ESI m/z 474,4 (100%, $[M-Cl]^+$); HRMS-ESI m/z 474,3949 ($[M-Cl]^+$, for $C_{28}H_{48}O_3N$ calculated 474,3947).

**Example 8**

**20-Oxo-5β-pregnan-3α-yl-2-(trimethylammonium) acetate hydrochloride**

Betain chloride hydrochloride was prepared from betaine (62 mg, 0.4 mmol) in dry dichloromethane (1 ml) under argon atmosphere. To the reaction mixture cooled in ice bath oxalyl chloride was added dropwise (0.5 ml; 5.82 mmol) followed by addition of catalytic amount of anhydrous dimethylformamide (3 μl; 0.03 mmol). Heterogenous mixture was let to reach room temperature and stirred for 16 h. During that time all solids were dissolved. Then the solvents were evaporated under reduced pressure and solid was dissolved in nitromethane (2 ml) and dry pyridine (3 μl; 0,03 mmol) in argon atmosphere. To the solution of chloride the compound II was added (100 mg; 0,31 mmol). Reaction mixture was stirred 4 h. The reaction was quenched by addition of water (10 ml). The mixture was acidified to pH 4 with 5% aq. HCl. Product was extracted with $CHCl_3$ (3 x 20 mL). Combined organic phase was dried with anhydrous $MgSO_4$ and evaporated under reduced pressure. Trituration with benzene removed the unreacted lipophilic starting material and the remaining product was subsequently crystallized from $CHCl_3$ : *n*-heptan, 1:1 to give white crystals (81 mg; 62%).

m.p. = 225-226°C (decomposition)

$[\alpha]_D$ = +97.8 (c 0.231)

$^1$H NMR (500 MHz, $CDCl_3$) δ 4,76-4,68 (m, 1H, 3-CH), 3,78 (d, 2H, 2'-$CH_2$), 3,49 (s, 9H, $NCH_3$), 2,53 (t, 1H, J = 9.0 Hz, 17-CH), 2,12 (s, 3H, 21-$CH_3$), 0,94 (s, 3H, 19-$CH_3$), 0,60 (s, 3H, 18-$CH_3$).
$^{13}$C NMR (101 MHz, $CDCl_3$) δ 209.53, 164.26, 77.75, 63.76, 63.28, 56.50, 54.22, 44.26, 41.84, 40.34, 39.04, 35.74, 34.85, 34.56, 31.92, 31.50, 26.80, 26.36, 26.15, 24.37, 23.13, 22.90, 20.83, 13.39.
IR ($CHCl_3$): 2956 ($NMe_3^+$), 1722 (C=O, ester), 1699 (C=O, keton), 1478 ($NMe_3^+$) 1386 ($CH_3$), 1360($COCH_3$), 1230 ($NMe_3^+$), 1188 (CO), $cm^{-1}$.
ESI m/z 418,3 (100%, $[M-Cl]^+$); HRMS-ESI m/z 418,3323 ($[M-Cl]^+$, pro $C_{28}H_{48}O_3N$ vypočteno 446,3319).

**Example 9**

**3α-Amino-5β-pregnan-20-one hydrochloride salt**

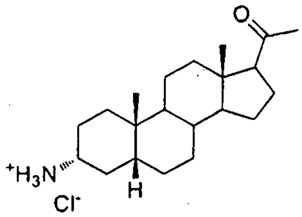

3-α-Azido-5β-pregnan-20-one (2.98 g; 8.68 mmol) was dissolved in MeOH (150 mL), 5% $Pd/CaCO_3$ catalyst was added to the solution and the steroid was hydrogenated for 3 hrs under a slight overpressure of hydrogen. After the conversion was complete, the catalyst was filtered off, the solvent was partially removed in vacuo, the residue was poured into 5% aq. HCl (400 mL) and extracted with $CHCl_3$ (5x50 mL). The organic phase was dried with $Na_2SO_4$ and evaporated on rotavap. Crystallization of the oily residue from $CHCl_3$:*n*-heptane afforded white crystals of salt 3α-amino-5β-pregnan-20-one hydrochloride salt (2.73 g; 89%).

m.p.= 276-277.5°C

$[\alpha]_D$ = +103.1 (c 0.258).

$^1$H NMR (400 MHz, $CDCl_3$) δ 0.59 (s, 3H, 18-$CH_3$); 0.96 (s, 3H, 19-$CH_3$); 2.11 (s, 3H, 21-$CH_3$); 2.55 (t, 1H, J = 8.9, 17-CH); 3.15-3.27 (bm, 1 H, 3-CH); 8.33 (bs, 3H, $NH_3^+$).
$^{13}$C NMR (101 MHz, $CDCl_3$) δ 209.37, 63.68, 56.28, 51.92, 44.21, 42.17, 40.34, 38.93, 35.81, 35.15, 34.62, 31.60, 31.46, 26.73, 26.29, 26.18, 24.43, 23.33, 22.95, 20.89, 13.39.
IR ($CHCl_3$): 1157, 1193 (CO), 1238, 1255 ($NMe_3^+$), 1359, 1386 ($CH_3$), 1451 ($COCH_3$), 1700(C=O, ketone), 1721 (C=O, ester), 2958 ($NMe_3^+$).
ESI m/z 318.4 (100%, $[M]^+$), 340.4 (17%, $[M-H+Na]^+$) ; HRMS-ESI m/z 318.27922 ($[M]^+$, $C_{21}H_{36}ON$ requires 318.27914).

**Example 10**

**3-(Trimethylammonium)-5β-pregnan-20-one chloride**

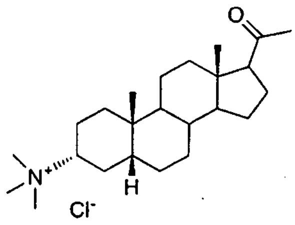

3α-Amino-5β-pregnan-20-one hydrochloride salt (200 mg; 0.57 mmol) was dissolved in MeOH (10 mL) and $NaHCO_3$ (285 mg; 3.39 mmol) was added to the solution. Finally, methyl iodide (0.22 mL; 3.53 mmol) was added to the stirred suspension and the mixture was refluxed for 3 days. The reaction mixture was then concentrated on rotavap, the reaction mixture was poured into 5% aqueous HCl (50 mL) and extracted with $CHCl_3$ (4x15 mL). The combined organic phase was washed with brine, dried over $Na_2SO_4$ and evaporated on rotavap. The solid residue was dissolved in minimal amount of MeOH and this solution was applied to a column of Amberlite IRA-400 (in $Cl^-$ phase; 1x20 cm; packed in MeOH) and eluted slowly with the same solvent. The fraction containing steroid was collected, evaporated and crystallized from $Et_2O$ to afford white crystals (151 mg; 67%).

m.p. = 237-240°C (decomposition);

$[\alpha]_D$ = +91.9 (c 0.235).

$^1H$ NMR (1H, J = 9.0, 17-CH); 3.42 (s, 9H, $NCH_3$) 3.66 (tt, 2H, $^2J_1$ = 12.1, $^2J_2$ = 3.3, 3-CH). $^{13}C$ NMR (101 MHz, CDCl3) δ 209.38, 75.46, 63.64, 56.25, 51.52, 44.18, 42.60, 40.74, 38.83, 35.71, 35.25, 34.41, 31.47, 27.00, 26.81, 26.24, 24.32, 22.95, 22.93, 21.39, 20.83, 13.40.

IR ($CHCl_3$): 2962 ($NMe_3^+$), 1699 (C=O, ketone), 1488 ($NMe_3^+$), 1386 ($CH_3$), 1359($COCH_3$). ESI m/z 360.3 (100%, $[M-Cl]^+$); HRMS-ESI m/z 360.32609 ($[M-Cl]^+$, $C_{24}H_{42}NO$ requires 360.32697).

**Example 11**

**Effects of pregnanolone sulfate and its cationic analogs on recombinant NMDA receptors**

*HEK293 cells* (American Type Culture Collection, ATTC No. CRL1573, Rockville, MD) were cultivated in Opti-MEM® I media (Invitrogen) with addition of 5% fetal bovine serum at 37 °C and transfected with NR1-1a/NR2B/GFP plasmids, as described in the scientific literature (Cais et al., 2008). Same amounts (0.3 μg) of cDNA coding NR1, NR2 and GFP (green fluorescent protein) (pQBI 25, Takara, Japan) were mixed with 0.9 μl of Matra-A Reagent (IBA, Göttingen, Germany) and added to confluent HEK293 cells cultivated in v 24-pit cultivating plate. After trypsination, the cells were re-suspended in Opti-MEM® I containing 1% fetal bovine serum. Subsequently, 20 $mmol.l^{-1}$ $MgCl_2$, 1 mmol $l^{-1}$ D,L-2-amino-5-phosphonopentanoic acid, 3 $mmol.l^{-1}$ kynurenic acid was added to the mixture and cells were inoculated on the polylysine-coated glass plates having 25 mm in diameter. The following genes coding NMDA receptor subunits were used for transfection: NR1-1a (GenBank accession no. U08261) and NR2B (GenBank accession no. M91562).

HEK293 Cultured cells were used for electrophysiological investigations with a latency of 16-40 h after transfection. Whole-cell currents were measured by patch-clainp amplifier (Axopatch 1D; Axon Instruments, Inc. Foster City, USA) after capacitance and serial resistance (<10 M'Ω) compensation to 80-90 %. Agonist-induced responses were filtered to 1 kHz (8-pole Bessel filter; Frequency Devices, Haverhill, USA), digitized with sampling frequency of 5 kHz and analyzed by pClamp version 9 software (Axon Instruments, USA). Micropipettes made of borosilicate glass were filled with intracellular solution, containing 125 $mmol.l^{-1}$ D-glukonic acid, 15 $mmol.l^{-1}$ cesium chloride, 5 $mmol.l^{-1}$ EGTA, 10 $mmol.l^{-1}$ HEPES buffer, 3 $mmol.l^{-1}$ magnesium chloride, 0.5 $mmol.l^{-1}$ calcium chloride and 2 $mmol.l^{-1}$ magnesium-salt of ATP (pH adjusted to 7.2 by cesium hydroxide solution). Extracellular solution (ECS) contained 160 $mmol.l^{-1}$ sodium chloride, 2.5 $mmol.l^{-1}$ potassium chloride, 10 $mmol.l^{-1}$ HEPES, 10 $mmol.l^{-1}$ glucose, 0.2 $mmol.l^{-1}$ EDTA a 0.7 $mmol.l^{-1}$ calcium chloride (pH adjusted to 7.3 by sodium hydroxide solution). Glycine was added to both testing and control solution. Moreover, bicuculline (10 $\mu mol.l^{-1}$) and tetrodotoxin (0.5 $\mu mol.l^{-1}$) was added to hippocampal cultures. Steroid-containing solutions were prepared from fresh solution (20 $mmol.l^{-1}$) of steroid dissolved in dimethyl-sulfoxide (DMSO). Same concentrations of DMSO were used in all extracellular solutions. Control and experimental solutions were applied via microprocessor-controlled perfusion system with approx. rate of solution exchange in areas adjacent to cells reaching ~10 ms.

Current responses produced by 100 μmol.l$^{-1}$ of NMDA (in the case of hipocampal neurones), or by 1 mmol.l$^{-1}$ of glutamate (on recombinant NMDA receptors) were measured at membrane potential maintained at -60 mV. Similarly as described before, pregnanolone sulfate decreased the amplitude of responses elicited by NMDA. After application of 100 μmol.l$^{-1}$ of pregnanolone sulfate the mean inhibition effect reached 71.3 ± 5.0 (n = 5) on hipocampal neurones, and ± 8.2 % (n = 5) on recombinant NR1/NR2B receptors (Petrovic et al., 2005, 25(37), 8439-50). Our synthetic analogs of pregnanolone sulfate exhibited significant inhibitory effect (fig.1) at concentrations 50, 100, or 200 μmol.l$^{-1}$ (chosen to reach maximal inhibition from 30 to 70%). Relative effect of steroid-induced inhibition was used for calculating $IC_{50}$. $IC_{50}$ was calculated using formula $RI = 1 - (1/1 + ([steroid]/IC_{50})^h)$, where *RI* denotes relative effect of steroid-induced inhibition and *h* is a parameter of Hill's coefficient (1.2). $IC_{50}$ values are stated in the following table.

Newly synthesized analogs from Examples 1-6 have the same mechanism of action on the NMDA receptors as pregnanolone sulfate, but they differ in their relative affinities (see Table 1).

Table 1

| Tested compound: **Compound from example** | Relative inhibition effect (%) | $IC_{50}$ (μmol) | Number of cells | Concentration (μmol.l$^{-1}$) |
|---|---|---|---|---|
| Pregnanolone sulfate | 67.2 ± 8,2 | 55 | 5 | 100 |
| Compound from example 2 | 69.2 ± 9,6 | 5.3 +/- 2,1 | 5 | 10 |
| Compound from example 6 | 65.4 ± 3,4 | 5.9+/-0,7 | 5 | 10 |
| Compound from example 5 | 79.2 ± 4.2 | 3.3 ± 0,7 | 5 | 10 |
| Compound from example 7 | 85.6 +/- 2.3 | 23.6+/- 3,5 | 5 | 100 |
| Compound from example 8 | 57.0 ± 9,6 | 22.4+/- 4,1 | 5 | 30 |
| Compound from example 10 | 69.2 ± 9,6 | 106.7+/- 12,1 | 5 | 100 |

The results show that synthetic analogs of pregnanolone sulfate has the same mechanism of action on NMDA receptors as pregnanolone sulfate; however, they differ in their affinity for these receptors. All experiments complied with standard accepted rules for animal care (Animal Protection Code of the Czech Republic, EU directives, and National Institute of Health guidelines).

## Example 12

### Effect of compound from example 2 (pregnanolone argininate) on spontaneous locomotor activity in the open-field test

For assessment of the spontaneous locomotor activity, animals (mice strain B6) were observed for 60 min in a circular open-field apparatus (diameter 82 cm). Animals were tracked with iTrack (Biosignal group, USA) and total path during this session was evaluated. The pregnanolone argininate was applied subcutaneously (dissolved in cyclodextrin) prior to behavioral observations at doses 10 mg/kg; control animals were injected with saline and cyclodextrin.

Results showed that pregnanolone argininate did not significantly alter the spontaneous locomotor activity. Both control groups exhibited similar total distance though there is apparent, yet insignificant, tendency of cyclodextrin to suppress locomotion. (see Fig. 2).

## Example 13

### Effect of compound from example 2 (pregnanolone argininate) and pregnanolone nitrate on locomotion and cognitive function in Carousel maze

Effect of pregnanolone argininate and nitrate and action of dizocilpine (a non-competitive antagonist of NMDA receptors; standardly used as positive control and referred hereafter as dizocilpine) on the behavior of rats in Carousel maze (active allothetic place avoidance (AAPA)), a spatial task requiring spatial orientation and cognitive functions

The effect of compounds on cognitive behaviors and locomotion was assessed using Carousel maze (active allothetic place avoidance (AAPA) task), which requires intact hippocampi and ability of animals to navigate in space

using distinct reference frames (spatial navigation and "cognitive coordination") (Wesierska et al., 2005) and it also allows measuring changes in the accompanying locomotor activity. AAPA task training involves animals trained to move over a slowly rotating uniform circular arena, on which a prohibited sector is defined, entering which is punished by a mild footshock. A shock sector and its position can be determined solely by its relationship to distal orienting cues in the room (Stuchlik et al., 2008).

This task is highly dependent upon hippocampal formation, with unilateral reversible ablation of this structure with TTX leading to avoidance deficit (Cimadevilla et al., 2001). Animals solving this task should walk in a direction opposite to arena rotation; otherwise it would be repeatedly brought to a fixed sector by arena rotation. We conducted 4 acquisition session of AAPA task, during which the sector location was always reinforced and remained stable throughout the training. Each session lasted 20 min.

The effect was tested on 2-month-old Long-Evans male rats. Control animals obtained cyklodextrine (s.c.), experimental groups received subcutaneous (s.c.) injections of the compound from example 2 at doses 1 mg/kg (dissolved in cyclodextrin). Intraperitonal application of dizocilpine (a non-competitive NMDA receptor antagonist; showing significant neuroprotective activity but exerting cognition-disturbing and psychotomimetic effects) was used as a positive control. Dizocilpine was applied at doses 0.15 mg/kg. It is worth noting that dizocilpine (albeit its experimental neuroprotective activity) is often used to model schizophrenia-like behaviors in humans and it has been repeatedly shown to exert a dose-dependent learning deficit and hyperlocomotion.

The results in four daily sessions are shown in Fig. 3. For clarity, statistical evaluations were performed for the final sessions (representing asymptotic levels of control animals); this approach has repeatedly proved useful in evaluation on neuropharmacological data from AAPA task.

Locomotor activity of animals was assessed in the AAPA as total distance traveled in the coordinate frame of the arena (without passive rotation) in each 20-min session. Animals treated with pregnanolone argininate and nitrate failed to exhibit either *decrease* or *increase* in total distance compared to controls, whilst dizocilpine stimulated the locomotor activity.

It should be emphasized that hyperactivity (hyperlocomotion) induced by antagonists of NMDA receptors is sometimes considered to be into certain extent analogous to human positive symptoms of schizophrenia as both phenomena has been shown to relate to hyperfunction of mesolimbic dopaminergic circuits. Evaluation of locomotor activity in the AAPA task has shown that pregnanolone argininate and nitrate did not alter locomotor activity in this behavioral configuration. The results of activity analysis are depicted in Fig. 3.

Fig 3. shows the total distance per session in the AAPA training, after application dizocilpine and pregnanolone argininate and nitrate. Neither drug caused significant alteration of locomotor activity when evaluated on session 4.

Number of entrances into shock sector

Another parameter, which can be measured and which brings a spatial aspect to behavioral analysis, is number of entrances into prohibited sector (occasionally termed "number of errors"). This parameter actually shows the overall spatial performance of the task, indicating "efficiency, in which can rats learn this task", and it is related to cognitive functions of animals. Results have shown that pregnanolone argininate and nitrate do not caused worsening in this parameter, whilst dizocilpine dose-dependently disrupted this parameter. Results of this experiment are shown in Fig. 4.

Fig. 4 illustrates the number of entrances (as a measure of cognitive functions) in every AAPA session after application of dizocilpine and pregnanolone argininate and nitrate. The dizocilpine led to impairment in this parameter whilst pregnanolone argininate and nitrate did not cause a statistical change in this parameter in comparison with controls. * denotes $p < 0.05$, *** denotes $p<0.001$ compared to cyclodextrin controls; statistical test performed for the final session (see above).

Taken together the results show no significant effect of compound from example 2 on locomotion activity and spatial cognition. On the other side MK-801, noncompetitive NMDA antagonist, disrupts a spatial cognition. Absence of serious side effect (typical for NMDA antagonists) of pregnanolone argininate and nitrate can be cleared up by mechanism of action (use-dependent action).

**Example 14**

**Anesthetic effect of compound from example 2 (pregnanolone argininate)**

Anesthetic effect of 3α5β pregnanolon argininate was tested on 2-month-old male mice strain B6. Animals were randomly assigned to 3 groups per 7 animals each. The first group was injected i.p. with saline, the second group received i.p. cyclodextrine, the third group received the pregnanolon argininate at a dose 100 mg/kg (dissolved in β-cyclodextrine).

Application of pregnanolon argininate lead to decreased locomotor activity (3-15 minutes after injection) decreased locomotor activity. Decreased locomotor activity gradually changed into anesthesia of animals (5-20 minutes after injection). Animals spontaneously awoke after 1-3 hours. Anesthetic effect related to the mechanism of action of this compound. Animals in controls groups did not display any behavioral and locomotion changes.

**Industrial applicability**

The compounds mentioned in the presented patent are industrially producible and applicable for treatment of numerous diseases of central nervous system, e.g. the following:

1) hypoxic and ischemic damage of the central nervous system, stroke, and other excitotoxicity-induced pathological alterations.
2) neurodegenerative changes and disorders
3) affective disorders, depression, PTSD and other stress-related diseases
4) schizophrenia and other psychotic disorders
5) pain, hyperalgesia and disorders of nociception
6) addictive disorders
7) multiple sclerosis and other autoimmune diseases
8) epilepsy and other seizure disorders
9) hyperplasic changes in CNS, CNS tumors including gliomas

**Literature cited**

1. Cais O., Sedláček M., Horak J., Dittert I, Vyklický ml. L. Temperature dependence of NR1/NR2B NMDA receptor channels. Neuroscience 2008, 151(2), 428-438.
2. Cimadevilla JM, Wesierska M, Fenton AA, Bures J; Inactivating one hippocampus impairs avoidance of a stable room-defined place during dissociation of arena cues from room cues by rotation of the arena. Proc. Natl. Acad. Sci. USA 98 (2001), 3531-3536.
3. Lindstedt G., Lindstedt S.; Studies on biosynhesis of carnitine. J. Biol. Chem. 1965, 240(1), 316-321.
4. Morrow AL.; Recent developments in the significance and therapeutic relevance of neuroactive steroids-Introduction to the special issue. Pharmacol. Ther. 2007, 116(1), 1-6.
5. Petrovic M., Sedlacek M., Horak M., Chodounska H., Vyklicky L. Jr.; 20-oxo-5beta-pregnan-3alpha-yl is a use-dependent NMDA receptor inhibitor. J Neurosci. 2005, 25(37), 8439-50.
6. Rambousek L., Bubenikova-Valesova V., Kacer P., Syslova K., Kenney J., Holubova K., Najmanova V., Zach P., Svoboda J., Stuchlik A., Chodounska H., Kapras V., Adamusova E., Borovska J., Vyklicky L., Vales K.; Cellular and behavioural effects of a new steroidal inhibitor of the N-methyl-D-aspartate receptor $3\alpha5\beta$-pregnanolone glutamate. Accepted for publication in Br. J. Pharmacol., DOI: 10.1111/j.1476-5381.2011.01816.x
7. Stuchlik A, Petrasek T, Vales K; Dopamine D2 receptors and alphal-adrenoceptors synergistically modulate locomotion and behavior of rats in a place avoidance task. Behav. Brain Res. 189 (2008), 139-144.
8. Wesierska M; Dockery C; Fenton AA; Beyond memory, navigation, and inhibition: behavioral evidence for hippocampus-dependent cognitive coordination in the rat. J. Neurosci. 25 (2005), 2413-2419.
9. Villmann C., Becker CM.; On the hypes and falls in neuroprotection: targeting the NMDA receptor. Neuroscientist. 2007, 13(6), 594-615.2.
10. Weaver CE., Land MB., Purdy RH., Ruchards KG., Gibbs TT., Farb DH.; J. Pharm. Exp. Ther. 293 (2000), 747.


**Claims**

**1.** Pregnanolone derivatives, substituted in the 3alpha-position with the cationic group, of general formula I

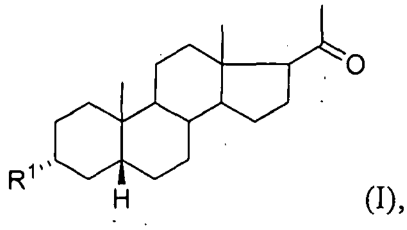

(I),

wherein $R^1$ represents the group of general formula $R^3$-$R^2$-C($R^{13}$)-$R^4$-, where $R^2$ means $(CH_m)_n$- group wherein m is 0 to 2, n is 1 to 18 which forms straight chain, which may be additionally substituted by one or more halogen atoms or primary, secondary or tertiary amino group, which may be either free or in case of primary amino group protected by a removable protecting group, chosen from *tert*-butoxycarbonyl, trityl, benzyloxycarbonyl, 9-fluorenylmethoxycarbonyl or *p*-nitrobenzyloxycarbonyl,
$R^3$ represents cationic group, chosen from guanidyl group of general formula (a)

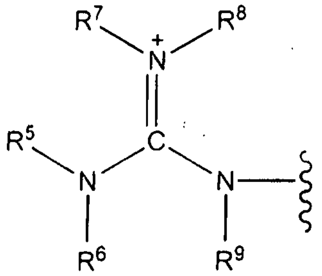

(a),

or ammonium group of general formula (b)

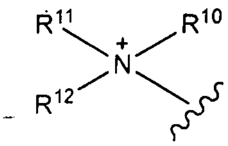

(b),

where $R^5$ - $R^{12}$ are hydrogen atoms or alkyl or alkenyl groups with 1 to 18 carbon atoms in a straight or a branched carbon chain, $R^{13}$ is chosen from a group, involving oxygen, nitrogen or sulfur atom bound by a double bond to carbon, or $R^{13}$ are two hydrogen atoms;
$R^4$ is bivalent or multivalent atom, chosen from oxygen, nitrogen or carbon atom and in case where $R^4$ is multivalent atom, that is carbon or nitrogen, its additional valence or valences are one or more hydrogens and any of hydrogen atoms may be substituted by alkyl or alkenyl having from 1 to 4 carbon atoms.

2. Pregnanolone derivatives, substituted in 3alpha-position with cationic group, of general formula I according to claim 1, for usage in treatment of neuropsychiatric disorders related to dysbalances of glutamatergic neurotransmitter system, as ischemic CNS injury, neurodegenerative changes, and disorders of central nervous system, mood disorders, depression, post-traumatic stress disorder, and other stress-related disorders, anxiety, schizophrenia, and other psychotic illnesses, pain, addiction, multiple sclerosis, epilepsy, and gliomas.

3. Pharmaceutical preparation containing pregnanolone derivatives, substituted in 3alpha-position with cationic group, of general formula I as active component.

4. Pharmaceutical preparation according to claim 3 for treatment of neuropsychiatric disorders related to dysbalance of glutamatergic neurotransmitter system, especially ischemic central nervous system injury, neurodegenerative changes and disorders of central nervous system, mood disorders, depression, post-traumatic stress disorder, and other stress-related disorders, anxiety, schizophrenia, and other psychotic illnesses, pain, addiction, multiple sclerosis, epilepsy, and gliomas.

5. The usage of pregnanolone derivatives, substituted in 3alpha-position with cationic group, of general formula I according to the claim 1, for production of standards of neuroprotectives and neuroleptics, or analytical standards utilized in experimental research, analytic chemistry; also utilization of these compounds as active substances contained in dietary supplements or compounds contained in food supplements or cosmetic preparations designated for improvement of reactions of particular parts of organisms on higher stress, namely oxidative, nutrient, or caused by free radicals, or by ageing.

6. Pregnanolone derivatives, substituted in the 3alpha-position with the cationic group, of general formula

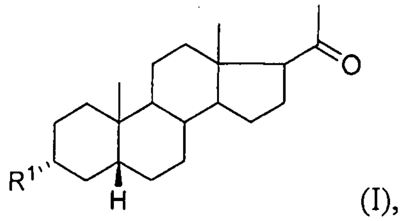

(I),

in which substiuent $R^1$ = ammonium group of general formula

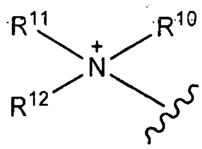

(b),

and $R^{10}$ - $R^{12}$ of this group are alkyl or alkenyl groups with 1 to 18 carbon atoms in a straight or a branched carbon chain.

**Patentansprüche**

1. Pregnanolon-Derivate der allgemeinen Formel I, die in der 3alpha-Position mit einer kationischen Gruppe substituiert sind,

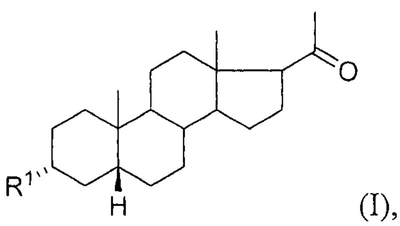

(I),

worin $R^1$ eine Gruppe der allgemeinen Formel $R^3$-$R^2$-C($R^{13}$)-$R^4$- ist,
in der $R^2$ eine $(CH_m)_n$-Gruppe bedeutet, worin m = 0 bis 2, n = 1 bis 18 ist, die eine lineare Kette bildett, die zusätzlich durch eine oder mehrere Halogenatome oder primäre, sekundäre oder tertiäre Aminogruppen substituiert sein kann, die entweder frei oder im Falle primärer Aminogruppen durch eine entfernbare Schutzgruppe geschützt sein kann, die aus *tert*-Butoxycarbonyl-, Trityl-, Benzyloxycarbonyl-, 9-Fluorenylmethoxycarbonyl- oder *p*-Nitrobenzyloxycarbonyl-Gruppen ausgewählt werden können,
$R^3$ eine kationische Gruppe ist, die aus einer Guanidinyl-Gruppierung der allgemeinen Formel (a)

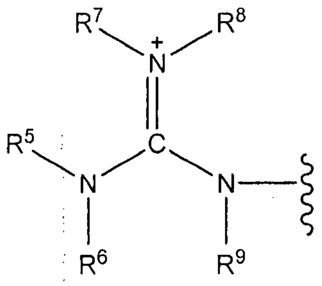

(a),

oder einer Ammonium-Gruppierung der allgemeinen Formel (b) ausgewählt werden kann

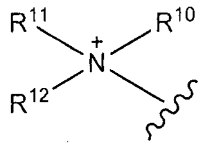

(b),

in denen $R^5$ - $R^{12}$ Wasserstoffatome oder Alkyl- oder Alkenylgruppen mit 1 bis 18 Kohlenstoffatomen in einer linearen oder verzweigten Kohlenstoffkette sind, $R^{13}$ wird aus einer Gruppe gewählt, an der ein Sauerstoff-, Stickstoff- oder Schwefelatom beteiligt ist, das mittels einer Doppelbindung an Kohlenstoff gebunden ist, oder $R^{13}$ sind zwei Wasserstoffatome;

$R^4$ ist ein bivalentes oder multivalentes Atom, das aus einem Sauerstoff-, Stickstoff- or Kohlenstoffatom ausgewählt werden kann und im Fall, dass $R^4$ ein multivalentes Atom, d.h. Kohlenstoff oder Stickstoff ist, seine zusätzliche Valenz oder die zusätzlichen Valenzen ein oder mehrere Wasserstoffatome sind, und jedes der Wasserstoffatome kann durch Alkyl- oder Alkenylgruppen einer Länge von 1 bis 4 Kohlenstoffatomen substituiert werden.

**2.** Pregnanolon-Derivate der allgemeinen Formel I entsprechend Patentanspruch 1, die in der 3alpha-Position mit einer kationischen Gruppe substituiert sind, zur Anwendung zur Behandlung neuropsychiatrischer Erkrankungen, die mit Dysbalancen des glutamatergen Neurotransmittersystems im Zusammenhang stehen, wie ischämische Verletzung des Zentralnervensystems, neurodegenerative Veränderungen und Erkrankungen des Zentralnervensystems, Gemütsstörungen, Depression, posttraumatischer Stress und andere stress-bedingte Störungen, Angstzustände, Schizophrenie und andere psychotische Erkrankungen, Schmerz, Abhängigkeit, multiple Sklerose, Epilepsie und Gliome.

**3.** Pharmazeutische Zubereitungen, die Pregnanolon-Derivate der allgemeinen Formel I, die in der 3alpha-Position mit einer kationischen Gruppe substituiert sind, als aktive Komponente enthalten.

**4.** Pharmazeutische Zubereitungen entsprechend Patentanspruch 3 zur Behandlung neuropsychiatrischer Erkrankungen, die mit Dysbalancen des glutamatergen Neurotransmittersystems im Zusammenhang stehen, besonders ischämische Verletzung des Zentralnervensystems, neurodegenerative Veränderungen und Erkrankungen des Zentralnervensystems, Gemütsstörungen, Depression, posttraumatischer Stress und andere stress-bedingte Störungen, Angstzustände, Schizophrenie und andere psychotische Erkrankungen, Schmelz, Abhängigkeit, multiple Sklerose, Epilepsie und Gliome.

**5.** Die Nutzung von Pregnanolon-Derivaten der allgemeinen Formel I entsprechend Patentanspruch 1, die in der 3alpha-Position mit einer kationischen Gruppe substituiert sind, zur Herstellung von Standards von Neuroprotektiva und Neuroleptika, oder analytischer Standards, die in der experimentellen Forschung, analytischen Chemie genutzt werden; weiterhin die Anwendung dieser Verbindungen als aktive Inhaltsstoffe in Nahrungsergänzungsmitteln oder als Verbindungen, die in Nahrungsergänzungsmitteln oder oder kosmetischen Präparaten enthalten sind, die auf die Verbesserung der Reaktion von bestimmten Teilen von Organismen gegenüber erhöhtem Stress gerichtet sind, und zwar oxidativem, Nährstoff-basiertem, durch freie Radikale verursachten oder durch Altern bedingtem.

6. Pregnanolon-Derivate der allgemeinen Formel I, die in der 3alpha-Position mit einer kationischen Gruppe substituiert sind,

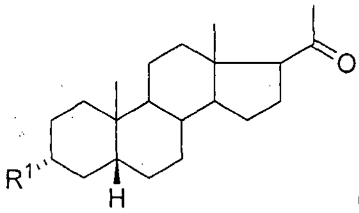

(I),

in der der Substituent $R^1$ eine Ammonium-Gruppe der allgemeinen Formel

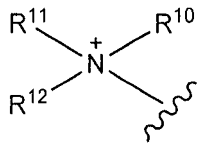

(b), ist

und $R^{10}$ - $R^{12}$ in dieser Gruppe Alkyl- oder Alkenylgruppen mit 1 bis 18 Kohlenstoffatomen in einer linearen oder verzweigten Kohlenstoffkette sind.

## Revendications

1. Dérivés de la prégnanolone, substitués en position 3alpha avec le groupe cationique, de formule générale I

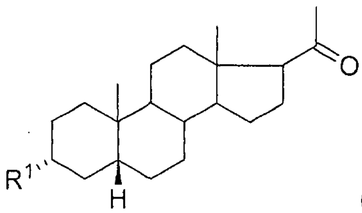

(I),

dans lequel $R^1$ représente le groupe de formule générale $R^3$-$R^2$-C($R^{13}$)-$R^4$-,
où $R^2$ est un groupe $(CH_m)_{n-}$ où m compris entre 0 et 2 et n compris entre 1 et 18,
qui forme une chaîne linéaire, qui peut être en outre substituée par un ou plusieurs atomes d'halogène ou groupe amino primaire, secondaire ou tertiaire, qui peut être libre ou dans le cas d'un groupe amino primaire protégé par un groupe protecteur amovible, choisi parmi tert-butoxycarbonyle, trityle, benzyloxycarbonyle, 9-fluorénylméthoxycarbonyle ou p-nitrobenzyloxycarbonyle,
$R^3$ représente le groupe cationique, choisi parmi le groupe guanidyle de formule générale (a)

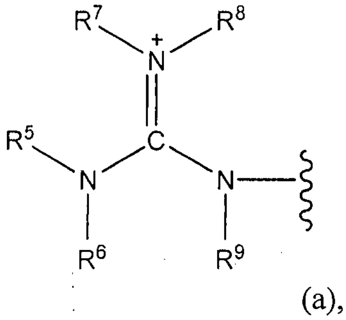

(a),

ou un groupe ammonium de formule générale (b)

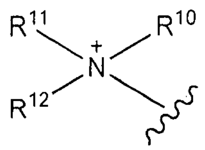

(b),

dans lequel $R^5$ - $R^{12}$ sont des atomes d'hydrogène ou des groupes alkyle ou alcényle ayant 1 à 18 atomes de carbone dans une chaîne carbonée droite ou ramifiée, $R^{13}$ est choisi dans un groupe comprenant un atome d'oxygène, d'azote ou de soufre lié par une double liaison carbone, ou $R^{13}$ sont deux atomes d'hydrogène;

$R^4$ est un atome bivalent ou multivalent choisi parmi l'atome d'oxygène, d'azote ou de carbone et dans le cas où $R^4$ est un atome multivalent, c'est-à-dire carbone ou azote, sa ou ses valences additionnelles sont un ou plusieurs hydrogènes où l'un des atomes d'hydrogène peut être substitué par un alkyle ou un alcényle ayant de 1 à 4 atomes de carbone.

**2.** Dérivés de la prégnanolone, substitués en position 3alpha avec le groupe cationique, de formule générale I, pour l'utilisation dans le traitement des troubles neuropsychiatriques liés aux déséquilibres du système neurotransmetteur glutamatergique, tels que lésions ischémiques du SNC, modifications neurodégénératives et troubles du système nerveux central, troubles de l'humeur, dépression, trouble du stress post-traumatique et autres troubles liés au stress, anxiété, schizophrénie et autres maladies psychotiques, douleur, toxicomanie, sclérose en plaques, épilepsie et gliomes.

**3.** Produit pharmaceutique contenant des dérivés de la prégnanolone substitués en position 3alpha avec le groupe cationique, de formule générale I, en tant que composant actif.

**4.** Produit pharmaceutique selon la revendication 3 pour le traitement des troubles neuropsychiatriques liés au déséquilibre du système neurotransmetteur glutamatergique, en particulier des lésions ischémiques du système nerveux central, des modifications neurodégénératives et des troubles du système nerveux central, des troubles de l'humeur, de la dépression, du stress post-traumatique et d'autres troubles liés au stress, anxiété, schizophrénie et autres maladies psychotiques telles que la douleur, la toxicomanie, la sclérose en plaques, l'épilepsie et les gliomes.

**5.** Utilisation de dérivés de pregnanolone, substitués en position 3alpha avec un groupe cationique, de formule générale I selon la revendication 1, pour la production d'étalons de neuroprotecteurs et de neuroleptiques, ou d'étalons analytiques utilisés dans la recherche expérimentale, la chimie analytique; utilisation également de ces composés comme substances actives contenues dans des compléments alimentaires ou des composés contenus dans des compléments alimentaires ou des préparations cosmétiques pour l'amélioration des réactions de parties particulières d'organismes sur un stress plus élevé, à savoir oxydant, nutritif, ou causé par des radicaux libres, ou par le vieillissement.

**6.** Dérivés de la prégnanolone, substitués en position 3alpha avec le groupe cationique, de formule générale

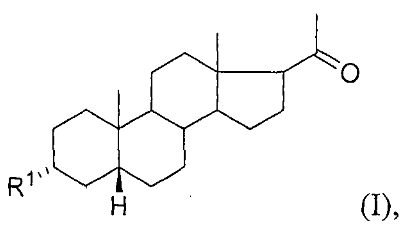

(I),

dans lequel substituant = le groupe ammonium de formule générale

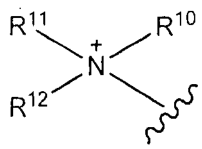

(b),

et $R^{10}$ - $R^{12}$ de ce groupe sont des groupes alkyle ou alcényle avec 1 à 18 atomes de carbone dans une chaîne carbonée linéaire ou ramifiée.

Fig. 1

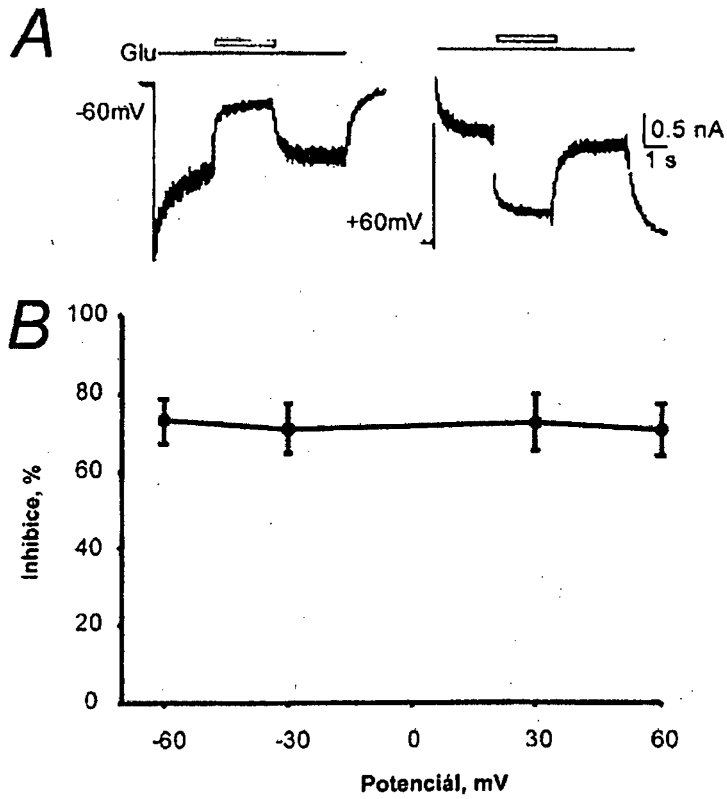
A
Glu
-60mV
+60mV
0.5 nA
1 s
B
100
80
60
40
20
0
Inhibice, %
-60
-30
0
30
60
Potenciál, mV

Fig. 2

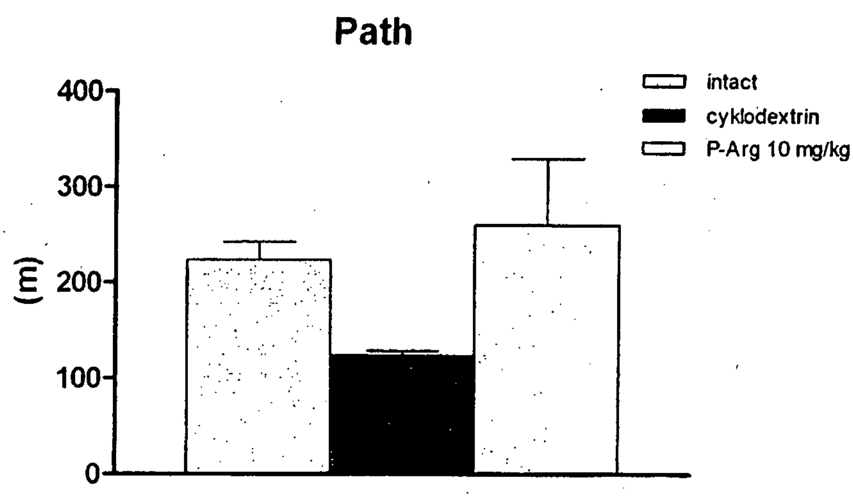
Path
400
300
200
100
0
(m)
intact
cyklodextrin
P-Arg 10 mg/kg

Fig. 3

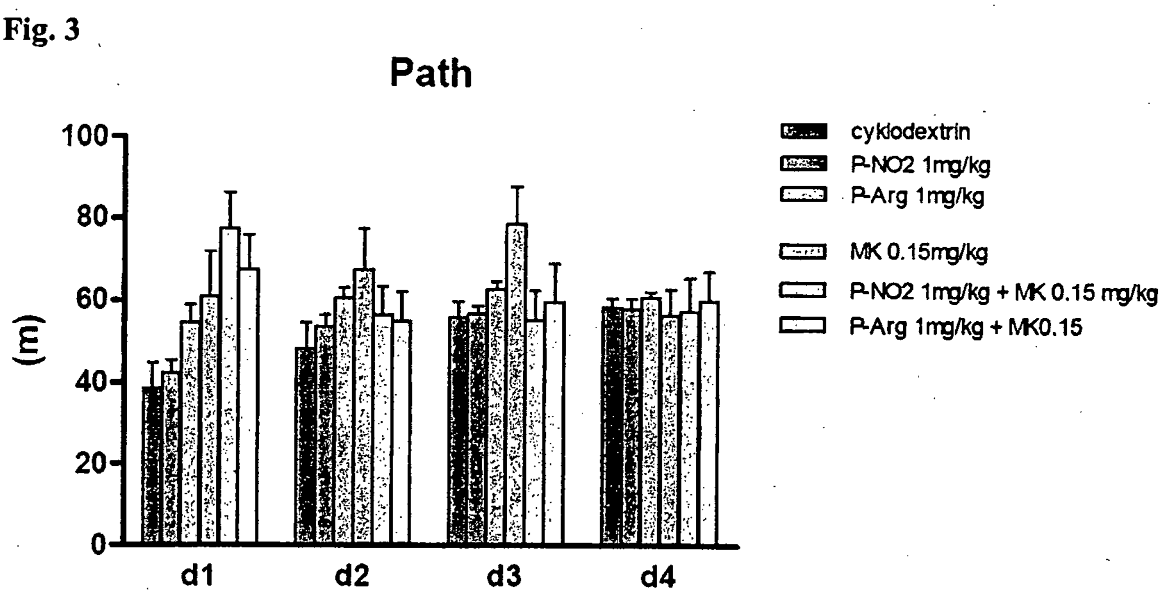
Path
100
80
60
40
20
0
(m)
d1
d2
d3
d4
cyklodextrin
P-NO2 1mg/kg
P-Arg 1mg/kg
MK 0.15mg/kg
P-NO2 1mg/kg + MK 0.15 mg/kg
P-Arg 1mg/kg + MK0.15

Fig. 4

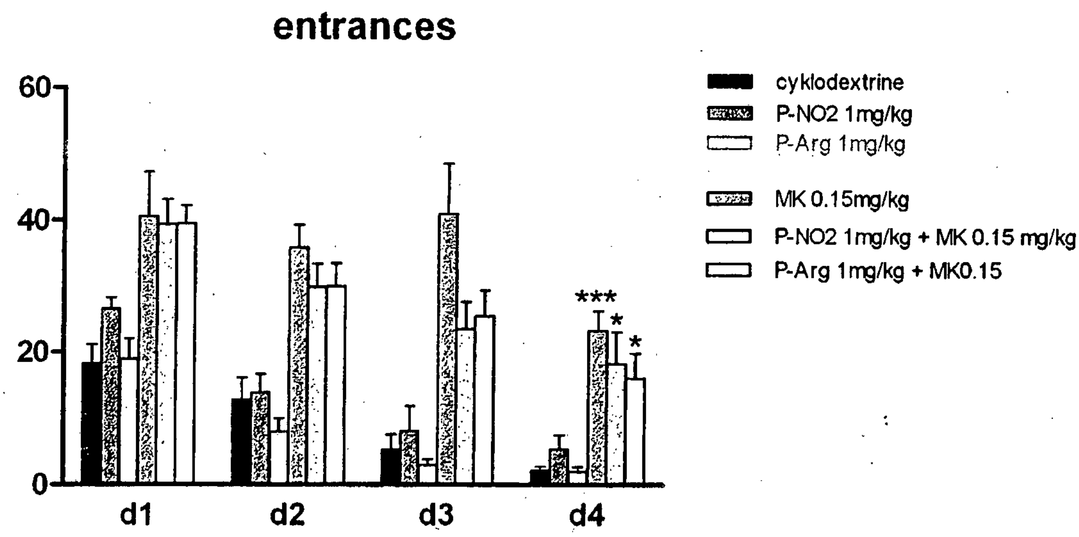
entrances
60
40
20
0
d1
d2
d3
d4
***
*
*
cyklodextrine
P-NO2 1mg/kg
P-Arg 1mg/kg
MK 0.15mg/kg
P-NO2 1mg/kg + MK 0.15 mg/kg
P-Arg 1mg/kg + MK0.15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009108804 A
- GB 962324 A

### Non-patent literature cited in the description


- **CAIS O. ; SEDLÁČEK M. ; HORAK J. ; DITTERT I ; VYKLICKÝ ML. L.** Temperature dependence of NR1/NR2B NMDA receptor channels. *Neuroscience,* 2008, vol. 151 (2), 428-438
- **CIMADEVILLA JM ; WESIERSKA M ; FENTON AA ; BURES J.** Inactivating one hippocampus impairs avoidance of a stable room-defined place during dissociation of arena cues from room cues by rotation of the arena. *Proc. Natl. Acad. Sci. USA,* 2001, vol. 98, 3531-3536
- **LINDSTEDT G. ; LINDSTEDT S.** Studies on biosynhesis of carnitine. *J. Biol. Chem.,* 1965, vol. 240 (1), 316-321
- **MORROW AL.** Recent developments in the significance and therapeutic relevance of neuroactive steroids-Introduction to the special issue. *Pharmacol. Ther.,* 2007, vol. 116 (1), 1-6
- **PETROVIC M. ; SEDLACEK M. ; HORAK M. ; CHODOUNSKA H. ; VYKLICKÝ L. JR.** 20-oxo-5beta-pregnan-3alpha-yl is a use-dependent NMDA receptor inhibitor. *J Neurosci.,* 2005, vol. 25 (37), 8439-50
- **RAMBOUSEK L. ; BUBENIKOVA-VALESOVA V. ; KACER P. ; SYSLOVA K. ; KENNEY J. ; HOLUBOVA K. ; NAJMANOVA V. ; ZACH P. ; SVOBODA J. ; STUCHLIK A.** Cellular and behavioural effects of a new steroidal inhibitor of the N-methyl-D-aspartate receptor 3α5β-pregnanolone glutamate. *Br. J. Pharmacol.*
- **STUCHLIK A ; PETRASEK T ; VALES K.** Dopamine D2 receptors and alphal-adrenoceptors synergistically modulate locomotion and behavior of rats in a place avoidance task. *Behav. Brain Res.,* 2008, vol. 189, 139-144
- **WESIERSKA M ; DOCKERY C ; FENTON AA.** Beyond memory, navigation, and inhibition: behavioral evidence for hippocampus-dependent cognitive coordination in the rat. *J. Neurosci.,* 2005, vol. 25, 2413-2419
- **VILLMANN C. ; BECKER CM.** On the hypes and falls in neuroprotection: targeting the NMDA receptor. *Neuroscientist.,* 2007, vol. 13 (6), 594-615
- **WEAVER CE. ; LAND MB. ; PURDY RH. ; RUCHARDS KG ; GIBBS TT. ; FARB DH.** *J. Pharm. Exp. Ther.,* 2000, vol. 293, 747